# EUROPÄISCHE PATENTSCHRIFT

(11) **EP 0 543 344 B1**
(45) Veröffentlichungstag und Bekanntmachung des Hinweises auf die Patenterteilung: **02.09.1998**
(21) Anmeldenummer: 92119635.8
(22) Anmeldetag: 17.11.1992
(51) Int. Cl.: C12N 15/65, C12N 15/52

(54) **Mikroorganismen zur Stabilisierung von Plasmiden**
Microorganismes for plasmid stabilisation
Microorganismes pour la stabilisation de plasmides

(30) Priorität: 21.11.1991 CH 3412/91
(43) Veröffentlichungstag der Anmeldung: 26.05.1993
(73) Patentinhaber: LONZA AG, CH-3945 Gampel/Wallis (CH)
(72) Erfinder: Zimmerman, Thomas, Dr., Naters (Kanton Wallis) (CH); Boraschi, Cristiana, Bioggio (Kanton Tessin) (CH); Burgdorf, Knut, Lalden (Kanton Wallis) (CH); Caubère, Cathérine, Nancy (FR)
(74) Vertreter: Weinhold, Peter, Dr.

(56) Entgegenhaltungen:
- EP-A- 0 178 764
- JOURNAL OF BACTERIOLOGY Bd. 165, Nr. 3, 1986, BALTIMORE US Seiten 856 - 863 Styrvold, Olaf B.; Falkenberg, Paal; Landfald, Bjarne; Eshoo, Mark W.; Bjoernsen, Tone; Stroem, Arne R. 'Selection , mapping, and characterization of osmoregulatory mutants of Escherichia coli blocked in the choline -glycine betaine pathway'
- JOURNAL OF BACTERIOLOGY Bd. 141, Nr. 2, 1980, BALTIMORE US Seiten 558 - 564 Atkinson, Katharine D.; Jensen, Bruce; Kolat, Anita I.; Storm, Elizabeth M.; Henry, Susan A.; Fogel, Seymour 'Yeast mutants auxotrophic for choline or ethanolamine'
- JOURNAL OF BACTERIOLOGY Bd. 113, Nr. 1, 1973, BALTIMORE US Seiten 218 - 233 White, Raymond F.; Kaplan, Louis; Birnbaum, Jerome 'Betaine-homocysteine transmethylase in Pseudomonas denitrificans, a vitamin B12 overproducer'

## Beschreibung

Die Erfindung betrifft neue Mikroorganismen, die mit einem neuen Hybridplasmid, enthaltend ein neues DNA-Fragment, transformiert sind, ein Verfahren zu deren Herstellung sowie die Verwendung dieser Mikroorganismen für einen Produktionsstamm mit stabilen Plasmiden.

Generell ist aus der Molekularbiologie bekannt, dass man zur Herstellung von bestimmten Verbindungen Mikroorganismen mit einem sogenannten "künstlichen" Plasmid transformiert, in das die Gene,die für diese bestimmte Verbindung codieren, eingebracht werden.
Ein besonderes Problem dieser Plasmide ist ihre Stabilität, d.h. ihre Eigenschaft, während der Zellteilung der Mikroorganismen nicht in kontrollierter Weise auf die Tochterzellen übertragen zu werden.
Dies führt dazu, dass während des Fermentationsverfahrens immer mehr Tochterzellen auftreten, die kein Plasmid bzw. weniger Plasmide enthalten.
Im Labormasstab kann man diesem Plasmidverlust derart entgegenwirken, indem man dem Kultivierungsmedium das Antibiotikum zuführt, dessen entsprechendes Antibiotika-Resistenzgen das Plasmid enthält. In Fermentationen im Grossmasstab hat sich jedoch die Zugabe des betreffenden Antibiotikums als nachteilig erwiesen. So zeigen beispielsweise einige Antibiotika, wie Tetracyclin ungünstige Effekte auf die Wachstums-, Teilungsund Fortpflanzungsfähigkeit Plasmid-enthaltender Mikroorganismen (Bioscience Reports, 5, 1985, S. 29 - 37; Gene, 39, 1985, S. 173 - 180). Ein weiterer Nachteil der Antibiotikum-Stabilisierung liegt darin, dass die Antibiotikum-Zugabe, insbesondere bei Fermentationen im Grossmasstab zu kostspielig ist. Desweiteren ist die Antibiotikum-Zugabe bei der Produktion von Pharmazeutika sowie bei der Produktion von Nahrungs- und Futterzusätzen unerwünscht oder unzulässig.

Eine weitere Methode, wie man diesem Plasmidverlust entgegenwirken kann, ist von H.Sakoda und T.Imanaka in J.Ferment. and Bioeng., Vol.69, 1990, S. 75 - 78, beschrieben.
Diese Methode beinhaltet ein stabiles "wirtsrekombinantes" Plasmidsystem, bei dem zunächst das Tryptophan-Operon im Chromosom des Wirts deletiert wird und damit die Wirtszelle inaktiv für den Tryptophan-Transport ist. Anschliessend wird die Wirtszelle mit einem rekombinanten Plasmid, welches dieses Tryptophan-Operon trägt, transformiert. Die Selektion der Wirtszellen mit diesem rekombinanten Plasmid erfolgt dann über den Tryptophan-Transport.

Die Nachteile dieser Methode liegen darin, dass trotz Selektion mit Tryptophan, aufgrund Diffusion im eigentlichen Fermentationsverfahren auch plasmidfreie Zellen wachsen können und damit zunehmend Tochterzellen auftreten, die keine Plasmide enthalten.

Die Aufgabe der vorliegenden Erfindung bestand darin, derartige Nachteile zu beseitigen und Mikroorganismen mit Plasmiden zur Verfügung zu stellen, deren Plasmide derart aufgebaut sind, dass sie sich mit einer billigen und leicht zugänglichen Substanz während des gesamten Fermentationsverfahrens stabilisieren lassen, wobei die gute Wachstums-, Teilungs- und Fortpflanzungsfähigkeit der Plasmid-enthaltenden Mikroorganismen gewährleistet sein muss.

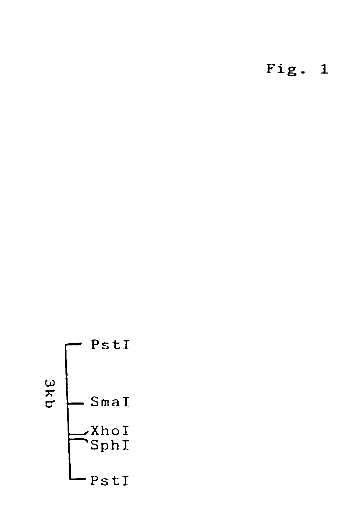

Diese Aufgabe konnte gelöst werden mit den erfindungsgemässen Mikroorganismen gemäss Patentanspruch 1, die sich dadurch auszeichnen, dass sie a) ein Hybridplasmid mit einem DNA-Fragment, enthaltend eine genetische Sequenz, die für die Verwertung von Betainen codiert und durch nachstehende Restriktionskarte (Fig. 1) charakterisiert ist: 
PstI
3kb
SmaI
XhoI
SphI
PstI
Fig. 1
 und b) eine Mutation im chromosomalen, für die Betain Verwertung codierenden Gen enthalten. Im folgenden werden unter Betainen Verbindungen verstanden, wie Betain, Cholin, Dimethylglycin und Sarcosin.

### Herstellung von Mikroorganismen mit stabilen Plasmiden

Die Herstellung der erfindungsgemässen Mikroorganismen erfolgt gemäss Patentanspruch 7, der nachstehend detaillierter erläutert wird. Die Herstellung erfolgt derart, dass man
in Schritt a)
   - I: Betain verwertende Mikroorganismen im Chromosom derart mutiert, dass sie nicht mehr befähigt sind, Betain zu verwerten
   - II: ein DNA-Fragment, enthaltend eine genetische Sequenz, die für die Verwertung von Betain codiert, isoliert
in Schritt b):
   - III: dieses isolierte DNA-Fragment in einen Expressions-Vektor einführt, wobei durch Ligation
   - IV: ein Hybridplasmid entsteht, dieses Hybridplasmid
in Schritt c):
   - V: mittels Transformation in den im Schritt I erhaltenen Mikroorganismus (Wirtsstamm) einbringt, wobei nach Selektion mit Betain Mikroorganismen mit, bezüglich der Betain-Verwertung, stabilen Plasmiden erhalten werden.
   - VI: Diese transformierten Mikroorganismen stellen einen Produktionsstamm mit bezüglich der Betain Verwertung stabilen Plasmiden dar, wenn deren Hybridplasmid ein zusätzliches für eine bestimmte Umsetzung codierendes Gen enthält.

### I Chromosomale Mutation von Betain verwertenden Mikroorganismen

Als Betain verwertende Mikroorganismen können erfindungsgemäss all jene dienen, die mit Betain bzw. Betainen als einzige Kohlenstoff-, Stickstoff- und Energiequelle wachsen.
Beispiele für solche Betain verwertende Mikroorganismen sind: Pseudomonas sp., Rhizobium-/Agrobacterium sp. oder Rhizobium sp..
Zweckmässig werden als Betain verwertende Mikroorganismen solche der Gattung Rhizobium - /Agrobacterium eingesetzt. Vorzugsweise wird der Mikroorganismus Rhizobium/Agrobacterium sp. HK1349 mit der DSM-Nr. 3944 angewendet. Dieser wurde am 4.11.1991 bei der Deutschen Sammlung für Mikroorganismen und Zellkulturen GmbH, Mascheroderweg 1b, D-3300 Braunschweig, hinterlegt.

Die Mutation des für die Betain Verwertung codierenden chromosomalen Gens, das im folgenden als beu bezeichnet wird, kann nach fachmännisch üblichen Methoden erfolgen. Beispiele für solche Mutationsmethoden sind:
Deletions-Mutation durch homologe Rekombination, Frame shift Mutation mit mutagenem Agens oder die Transposon-Insertionsmutation. Zweckmässig wird das Gen beu, im Falle von Rhizobium/Agrobacterium sp. HK1349, mittels der Methode der homologen Rekombination gezielt aus dem Mikroorganismen-Chromosom deletiert.

Hierzu wird zunächst ein DNA-Fragment, welches das Gen beu enthält aus dem Mikroorganismen-Chromosom isoliert und mit sogenannten "Hilfs"-Plasmiden in Mikroorganismen kloniert. Aus diesen "Hilfs"-Plasmiden wird dann der gewünschte für das beu codierende DNA-Abschnitt, dessen Isolation und Identifikation nachfolgend in II) beschrieben wird,deletiert.
Mit diesem sogenannten deletierten "Hilfs"-Plasmid kann dann mit fachmännisch üblichen Methoden eine entsprechende Deletion, über den Austausch mittels homologerRekombination, chromosomal eingeführt werden (Mol. Gen. Genet., 210, 1987, S. 381 - 381; J.Bacteriol., 171, 1989, S. 4617 - 4622).

Zweckmässig werden die Mikroorganismen Rhizobium-/Agrobacterium sp. HK1349 (DSM 3944) mittels der Methode der Deletionsmutation durch homologe Rekombination in die mutierten beu-inaktiven (Beu⁻) Mikroorganismen HK1349.4 überführt.

### II Isolation des DNA-Fragments beu

Als Quelle für das DNA-Fragment beu können die in I) bereits beschriebenen Mikroorganismen dienen.
Vorzugsweise dienen als Quelle für das DNA-Fragment beu die Mikroorganismen Rhizobium-/Agrobacterium sp. HK1349 mit der DSM-Nr. 3944, die wie bereits beschrieben hinterlegt sind.

Zur Isolation wird zweckmässig zunächst ein DNA-Fragment, enthaltend eine genetische Sequenz, die für die Verwertung von Betainen codiert, auf dem Chromosom von Rhizobium-/Agrobacterium HK1349 lokalisiert.
Die Lokalisation erfolgt mit fachmännisch üblichen Methoden, wie beispielsweise durch Erzeugung einer Transposon-Insertionsmutante im entsprechenden Mikroorganismen-Chromosom. Dabei wird das gesuchte DNA-Fragment beu mit einem Transposon, beispielsweise mit Tn5, markiert.
Die Identifizierung der entsprechenden Mutante mit diesem markierten DNA-Fragment kann dann über die Nichtverwertung von Betainen als Kohlenstoff-, Stickstoff- und Energiequelle erfolgen.

Die chromosomale DNA der identifizierten Transposon-Insertionsmutante wird dann zweckmässig mit dem Restriktionsenzym EcoRI geschnitten. Die dabei erhaltenen Fragmente werden mittels fachmännisch üblichen Methoden via Plasmide in E.coli kloniert.
Die dadurch erhaltenen und bezüglich Transposon-Antibiotika-Resistenz selektionierten Hybridplasmide besitzen ein aus HK1349 erhaltenes, mit Transposon markiertes EcoRI-DNA-Fragment mit einer Grösse von 18,2 kb (12,5 kb und 5,7 kb für Transposon Tn5).

Zur eigentlichen Isolation des intakten (nicht mit Transposon-markierten) DNA-Fragments beu wird zunächst die DNA von Rhizobium-/Agrobacterium HK1349 nach fachmännisch üblichen Methoden isoliert. Dann wird die isolierte DNA zweckmässig mit dem Restriktionsenzym EcoRI vollständig verdaut und aufgetrennt. Anschließend werden die EcoRI-DNA-Fragmente mit einer Grösse im Bereich von 12,0 bis 13,0 kb nach fachmännisch üblichen Methoden in E.coli kloniert. Über "Patch-Mating" Konjugation der verschiedenen Klone mit der beu-negativen Transposon-markierten Mutante können dann die Klone, die das intakte Gen beu enthalten, durch Komplementation der Mutation erkannt und isoliert werden. Das gesuchte Hybridplasmid liegt dann in E.coli vor.
Anhand von Komplementationstests mit Subklonen (Klone, die Deletionen in verschiedenen Bereichen des EcoRI-DNA-Fragments aufweisen) an der Transposon-Mutante wird ein 3 kb grosses PstI-geschnittenes DNA-Fragment, enthaltend eine genetische Sequenz, die für die Verwertung von Betainen codiert, identifiziert und isoliert.
Dieses DNA-Fragment ist Bestandteil der Erfindung und wird durch nachstehende Restriktionskarte (Fig. 1) charakterisiert

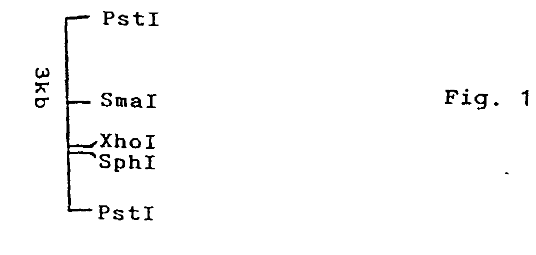
3kb
PstI
SmaI
XhoI
SphI
PstI
Fig. 1

Dieses DNA-Fragment ist im Hybridplasmid pLO32 enthalten und im Mikroorganismus HK1349.4 hinterlegt (DSM-Nr. 6712).

### III.Ligation des DNA-Fragments beu in Expressionsvektoren

Das so erhaltene DNA-Fragment beu kann mittels üblichen molekularbiologischen Techniken mit einer zuvor gleichermassen geschnittenen Expressionsvektor-DNA zu einem Hybridplasmid ligiert werden.

Expressionsvektoren enthalten üblicherweise einen geeigneten Promotor (Expressionskontrollsequenz). Hinter diesem Promotor liegen günstigerweise in Transkriptionsrichtung eine oder mehrere singuläre Schnittstellen für Restriktionsenzyme. In diese Schnittstellen wird dann üblicherweise der gewünschte Genabschnitt inseriert, an dessen Expression man interessiert ist.

Für die erfindungsgemässen Hybridplasmide können Expressionsvektoren mit breitem Wirtsspektrum ("broad host range") angewendet werden. Beispiele für solche Expressionsvektoren sind:
pKT240 (Gene, 26, 1983, S.273-282)
pME285 (Gene, 36, 1985, S.27-36)
pVK100 (Plasmid, 8, 1982, S.45-54)
Zweckmässig wird für die erfindungsgemässen Hybridplasmide der Expressionsvektor pKT240 mit den Restriktionsenzymen PstI geschnitten und die entstandenen Restriktionsenden mit dem DNA-Fragment beu mittels beispielsweise T4-DNA-Ligase ligiert.

### IV. Hybridplasmide

Die Erfindung betrifft weiterhin die so entstandenen Hybridplasmide, die das DNA-Fragment beu enthalten.
Grundsätzlich sind alle Hybridplasmide geeignet, welche in dem gewählten Mikroorganismus replizieren und das DNA-Fragment beu exprimieren können.
Um eine effektive Expression in einem Hybridplasmid zu erreichen, wird das DNA-Fragment beu in Transkriptionsrichtung zum Promotor angeordnet.
Besonders geeignet ist das Hybridplasmid pLO32, bestehend aus dem DNA-Fragment beu und aus dem Expressionsvektor pKT240, bei dem das DNA-Fragment beu in Transkriptionsrichtung zum Promotor P_{bla} (verantwortlich für die Ampicillin-Resistenz) angeordnet ist.
Dieses Hybridplasmid wurde am 17.9.1991 bei der Deutschen Sammlung für Mikroorganismen und Zellkulturen GmbH, Mascheroderweg 1b, D-3300 Braunschweig, im Mikroorganismus HK1349.4 hinterlegt (DSM-Nr. 6712).

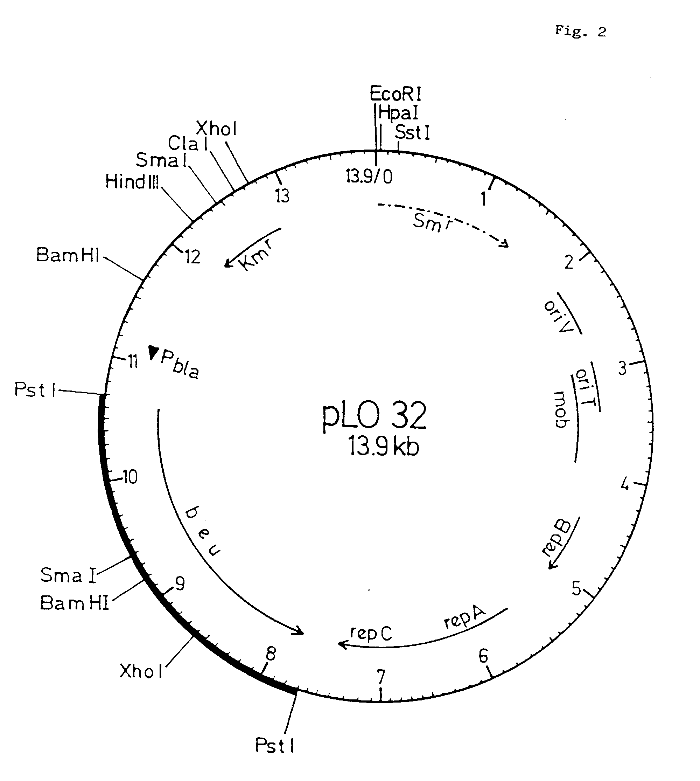

Fig. 2 zeigt ein Schema des Hybridplasmids pLO32.

### V. Transformation

Mit den so erhaltenen Hybridplasmiden werden die im Schritt

### I) erhaltenen Mikroorganismen transformiert.

Diese transformierten Mikroorganismen sind ebenfalls Bestandteil der Erfindung.

Die Transformation der Mikroorganismen mit den erfindungsgemässen Hybridplasmiden erfolgt nach bekannten Verfahren. Die Isolation bzw. Selektion der transformierten Mikroorganismen erfolgt auf einem selektiven Nährmedium, dem Betain als C-oder N-Quelle zugesetzt wird. Wenn, wie bevorzugt, Hybridplasmid pLO32 eingesetzt wird, erfolgt die Isolation bzw. Selektion der transformierten Mikroorganismen auf einem Nährmedium, dem Betain als C- oder N-Quelle zugesetzt wird. Vorzugsweise werden, nach Transformation, die Mikroorganismen HK1349.4 mit dem Hybridplasmid pLO32 (DSM-Nr. 6712) erhalten.

Zur Stabilisierung dieser transformierten Mikroorganismen setzt man zweckmässig dem Kultivierungsmedium Betaine in einer Konzentration von 0,2 bis 0,4.% zu. Als Kultivierungs- medium können die in der Fachwelt üblichen dienen, wie beispielsweise ein Mineralsalzumedium nach Kulla et al., Arch. Microbiol., 135, 1983, S. 1 - 7.

### VI. Produktionsstämme mit bezüglich der Betain Verwertung stabilen Plasmiden

Die Erfindung betrifft sowohl die Verwendung des DNA-Fragments beu zur Herstellung von bezüglich der Betain Verwertung stabilen Plasmiden als auch die Verwendung der nach der Transformation mit diesem stabilen Plasmid erhaltenen Mikroorganismen zur Herstellung von Produktionsstämmen mit stabilen Plasmiden. Demgemäss sind auch die resultierenden Produktionsstämme Bestandteil der Erfindung.

Diese Produktionsstämme sind erhältlich durch Transformation von Mikroorganismen enthaltend eine Mutation im chromosomalen, für die Betain Verwertung codierenden Gen, mit einem Hybridplasmid,das zusätzlich zum DNA-Fragment beu ein für eine bestimmte Umsetzung codierendes Gen enthält.

Diese stabilen Plasmide, enthaltend beu und das zusätzliche Gen, können nach fachmännisch üblichen Methoden erhalten werden, entweder
- z. B. durch Ligation des zusätzlichen Gens in ein Hybridplasmid enthaltend beu, oder
- z. B. durch Ligation des DNA-Fragments beu in ein Hybridplasmid, das bereits ein für eine bestimmte Umsetzung codierendes Gen enthält.

Zweckmässig wird, wenn ein Hybridplasmid verwendet wird, das bereits das zusätzliche Gen enthält, dieses mit dem Restriktionsenzym PstI geschnitten (linearisiert) und dann mit den PstI-DNA-Fragment beu zum stabilen Plasmid ligiert.

Wenn das zu verwendende Hybridplasmid das DNA-Fragment beu bereits enthält, erfolgt die Linearisierung zweckmässig an Restriktionsschnittstellen, die auch das zusätzliche Gen "flankieren". Der entsprechend geschnittene Vektor mit beu und das zusätzliche DNA-Fragment werden religiert.

Nach erneuter Transformation in die erfindungsgemässen Mikroorganismen, können diese während der Kultivierung mit Betainen die gewünschte Umsetzung ohne Plasmidverlust gewährleisten.

Vorzugsweise wird als stabiles Plasmid das Hybridplasmid pLO32, enthaltend ein zusätzliches für eine bestimmte Umsetzung codierendes Gen, verwendet. Dieses stabile Plasmid entspricht im wesentlichen dem Hybridplasmid pLO32. Vorzugsweise wird dann dieses stabile Plasmid in den Mikroorganismus HK1349.9 transformiert.

Als Beispiel für einen Produktionsstamm mit stabilen Plasmiden dient der Mikroorganismus HK1349.4 enthaltend als stabiles Plasmid, das Plasmid pLOL01. Zur Herstellung von pLOL01 wird das bereits in der EP-A 0477 828 beschriebene Hybridplasmid pL03, welches aus dem Expressionsvektor pKT240 und den Genen xylMA (codieren für das Enzym Xylol-Monooxygenase) besteht, mit dem Restriktionsenzym PstI linearisiert und dann mit dem DNA-Fragment beu ligiert. Dieses Plasmid entspricht im wesentlichen dem Hybridplasmid pLO32, da es im Unterschied zu pLO32 nur die zusätzlichen xylMA Gene enthält.

Nach Transformation von pLOL01 in den Mikroorganismus HK1349.4 ist dieser Produktionsstamm befähigt 2,5-Dimethylpyrazin in 5-Hydroxymethylpyrazin in Gegenwart von Betain ohne PlasmidVerlust zu überführen.

### Beispiel 1:

### Erzeugung einer Transposon (Tn5 )-Insertionsmutante und ihre phänotypische Identifizierung

Der Stamm Agrobacterium/Rhizobium sp. HK1349 (DSM 3944) wurde durch Selektionsdruck zur Entwicklung einer Spontanresistenz gegenüber Streptomycin (1000 µg/ml) gebracht. Diese Resistenz war nachweislich über 50 Generationen ohne Selektion stabil und wurde als Selektionsmarke verwendet.
0,2 ml einer Tn5-Donorkultur, E.coli S17-1/pSUP 2021 (Neomycin-resistent; R.Simon et al., Biotechnology 1 (1983), S. 784 - 790) wurden mit 2 ml der Rezipientenkultur HK1349 vermischt und zentrifugiert.
Die Zellen wurden in 0,9%iger Saline (NaCl-Lösung) gewaschen und in 100 µl 0,9% Saline resuspendiert. Die Konjugation des Rezipientenstammes mit dem Donorstamm erfolgte über Nacht bei 30°C auf trockenem Nutrientagar. Anschliessend wurden die Zellen geerntet und in Verdünnungen auf nachstehendem Selektionsmedium für Rezipient und Transposon plattiert.
Tn5-Mutanten von HK1349 wurden durch Selektion auf Nutrientagar mit Streptomycin (1000 µg/ml) und Neomycin (100 µg/ml) erhalten. Phänotypische Identifizierung gelang über Nichtverwertung von Betainen als C- oder N-Quelle in Mineralsalzmedium, (Kulla et al., Arch. Microbiol., 135, 1983, S. 1 - 7).

### Beispiel 2:

### Klonierung des Tn5-markierten DNA-Fragment aus dem HK1349-Genom

Nach bekannter Methode isolierte chromosomale DNA (J.Mol. Biol., 130, 1979, S.161-173) von Tn5-mutiertem HK1349 (5 µg) wurde vollständig mit EcoRI (4 Units/µg) verdaut. 2,5 µg Plasmid pBR325 (Gene, 2, 1977, S.95-113) wurde nach vollständiger Verdauung durch EcoRI (1 Unit/µg) mit alkal. Phosphatase (0,1 Unit/1-20 pmol DNA termini) behandelt (dephosphoryliert).
Rekombinante Hybridplasmide wurden unter Vermischen der genomischen DNA und pBR325 mit T4-DNA-Ligase (0,2 Units/µg DNA) in 400 µl Ligationspuffer (20 mM Tris-HCl, pH 7,2, 10 mM Dithioerythritol (DDT), 10 mM MgCl₂, 0,6 mM Adenosintriphospat (ATP) erhalten.
Die Inkubation erfolgte über Nacht bei 12°C. Aliquots des Ligationsgemisches wurden im Transformationsexperiment nach Cohen et al. (Proc.Natl.Acad.Sci., USA 96 (1972), S.2110-2114) mit E.coli ED8654 eingesetzt.
Selektioniert wurden die Transformanten auf ihre Resistenz gegen Ampicillin (100 µg/ml, pBR325) und Kanamycin (25 µg/ml, Tn5-markiertes Insert) auf Nutrientagar.
Alle klonierten Hybridplasmide trugen ein HK1349-Insert (12,5 kb + 5,7 kb für Tn5), welches mit Tn5 markiert war.
Eine genaue Restriktionskartierung belegte die Transposoninsertion im gleichen genomischen Fragment an der gleichen Stelle entsprechend identischem Phänotyp Beu⁻ (inaktives beu-Gen durch Tn5-Insertion; Genotyp beu) der selektionierten Tn5-Mutanten. Diese Mutanten werden im folgenden als HK4V11 bezeichnet.

### Beispiel 3:

### Klonierung des DNA-Fragments beu (unmarkiert) aus dem HK1349-Genom

HK1349-DNA wurde entsprechend zu Beispiel 2 isoliert und vollständig mit EcoRI (4 Units/µg) verdaut und über Agarose-Gelelektrophorese aufgetrennt. Die DNA-Fragmente im Grössenbereich von 12,0 kb bis 13,0 kb (markiertes Fragment besass die Grösse 12,5 kb) wurden aus Agarose-Elektrophorese-Gelen isoliert. Die isolierte DNA wurde entsprechend zu Beispiel 2 mit EcoRI geschnitten und mit dem entsprechend zu Beispiel 2 dephosphorylierten Vektor pVK100 (Plasmid 8 (1982), S. 45 - 54) ligiert. Aliquots des Ligationsgemisches wurden entsprechend zu Beispiel 2 im Transformationsexperiment in E.coli S17-1 (Biotechnology, 1, 1983, S. 1784 - 1791) eingesetzt.
Die Transformanten wurden auf ihre Resistenz gegen Tetracyclin (25 µg/ml) und Kanamycin (25 µg/ml) auf Nutrientagar selektioniert.
Die erhaltenen Transformanten wurden mittels "patch-mating"
Konjugation mit der Transposonmutante HK4V11 (beu) als Rezipientenstamm auf Insertion des gesuchten DNA-Abschnittes mit dem Gen beu überprüft: Antibiotika-resistente Transformanten wurden in festgelegtem Muster auf Selektionsmedium (Nutrientagar mit Kanamycin 2 µg/ml) beimpft. Parallel wurden Nutrientagarplatten mit einem Rasen des Rezipientenstammes HK4V11 beimpft.
Zur Konjugation wurden die Transformanten als Einzelklone auf den gewachsenen Zellrasen des Rezipientenstammmes gestempelt und über Nacht bei 30°C inkubiert.
Die "Mating"-Platten wurden schliesslich zur Selektion der erhaltenen Transkonjuganten auf dem im Beispiel 1 zitierten Mineralsalzmedium mit Betain (0,2 Gew.%), als Substrat, welches Donor und Rezipient nicht verwerten können, überstempelt. Bei den wachsenden Transkonjuganten wird der mutierte genomische DNA-Abschnitt des Rezipienten (HK4V11) durch Aufnahme eines Hybridplasmides mit dem entsprechenden intakten DNA-Bereich aus dem Donorstamm komplementiert (bzw. homolog rekombiniert). Komplementierende Hybridplasmide erhielten die Bezeichnung pVK100s. Zur Unterdrückung von Revertanten des Stammes HK4V11 wurde dem Medium Neomycin (100 µg/ml) zugegeben. Durch Hybridisierung gegen die klonierten, Tn5-markierten DNA-Fragmente konnte die erfolgreiche Klonierung des komplementierenden Fragmentes mit dem intakten beu-Gen aus dem HK1349 Genom auf dem Hybridplasmid pVK100s bestätigt werden.

### Beispiel 4:

### Identifikation des für beu-codierenden DNA-Subfragments

Durch Deletions-Klonierungen mit verschiedenen Restriktionsenzymen (BglII, XhoI, SphI, PstI) an dem Hybridplasmid pVK100s und anschliessende Komplementationen an der beu Tn5-Mutante HK4V11 konnte ein 3 kb grosser PstI-geschnittener DNA-Abschnitt auf dem Plasmid identifiziert werden, welcher für die Betain Verwertung codiert.
Dieser Abschnitt ist durch nachstehende Restriktionskarte (Fig. 1) charakterisiert.

3kb
PstI
SmaI
XhoI
SphI
PstI
Fig. 1

### Beispiel 5:

### Stabile Mutation von beu im HK1349-Genom

Da prinzipiell eine Tn-Insertionsmutation mit und ohne Antibiotika-Selektion sehr instabil ist, wurde dem Stamm HK1349 eine stabile Deletionsmutation eingeführt.
Dazu wurde nach üblichen Methoden mittels homologer Rekombination verfahren:
Das in Beispiel 3 erhaltene 12,5 kb grosse EcoRI-Fragment wurde in den nicht in HK-Stämmen exprimierenden Suizid-Vektor pACYC184 (J.Bacteriol., 134, 1978, S. 1141 - 1156) einkloniert und daraus das beu codierende, 3 kb grosse PstI-Fragment durch Restriktion mit PstI (1 Unit/µg) deletiert. Die Religation erfolgte über Nacht mit 1 Unit/µg T4-DNA-Ligase.
Das Deletionshybrid pCC6 wurde in E.coli HB101/pRK2013 (Helferplasmid zur Mobilisierung von pCC6) transformiert und konnte daraus mittels konjugativem Transfer in HK1349 eingeschleust werden. Die erhaltenen Transkonjuganten wurden gegen die Auxotrophie des Donors (Pro⁻ ; Prolin-negativ) und auf Antibiotika-Resistenz des Plasmids (Mineralsalzmedium 0,4% Glucose und Tetracyclin (25 µg/ml)) selektioniert.
Nur Zellen welche das Plasmid über homologe Rekombination chromosomal integriert haben, besitzen Tetracyclin-Resistenz und können auf dem Medium wachsen.
Um über ein zweites Rekombinationsereignis Vektor pACYC184 und intaktes beu-Gen aus dem HK1349-Chromosom wieder zu entfernen wurden diese Transkonjuganten im gleichen Medium ohne Selektion durch Tetracyclin über 100 Generationen kultiviert.
Um dann die Anzahl Tetracyclin-sensitiver beu-Mutanten zu erhöhen, wurde eine Selektion gegen den integrierten Vektor pACYC184 und das intakte beu-Gen durchgeführt:
Hierzu wurden die Zellen in 25 ml Komplexmedium NYB (Oxoid, Wesel, BRD) Tetracyclin (10 µg/ml) aufgenommen und für 6 h bei 30°C inkubiert.
Danach wurde zum Abtöten der wachsenden (Tetracyclinresistenten) Zellen 0,5 mg/ml D-Cycloserin und 15 mg/ml Penicillin G der Kultur zugegeben.
Nach weiterer Inkubation bei 30°C für 84 h wurden die Zellen zentrifugiert, dreimal in frischem NYB gewaschen und in geeigneter Verdünnung auf Nutrientagar plattiert.
18% der erhaltenen Kolonien waren Tetracyclinsensitiv, wovon ein Drittel gleichzeitig auch negativ in der Verwertung von Betain war. Die korrekte Einführung der Deletion von beu wurde über Hybridisierung gegen das 12,5 kb Fragment in Hybridplasmid pVK100s (aus Beispiel 3) bestätigt. (Nur ein um 3 kb verkürztes EcoRI-Fragment wurde markiert.)
Die resultierende Mutante HK1349.4 konnte durch das in geeignete Vektoren klonierte 3 kb grosse PstI-Fragment (beu) komplementiert werden. Die Vektoren sind in nachstehendem Beispiel 6 beschrieben.

### Beispiel 6:

### a) Klonierung des beu-Gens in diverse "broad host range-Vektoren"

In die bekannten "broad host range-Expressions-Vektoren" pKT240, pME285, pVK100 wurde das 3 kb grosse PstI-Fragment, welches für beu codiert, kloniert.
(Current Protocols in Molecular Biology, John Wiley and Sons, New York (1989), Abschnitt 3.16, Subcloning of DNA-Fragments). Dabei spielte die korrekte Orientierung des Inserts zum Promotor eine Rolle. Im Falle von pKT240 spielte dabei die korrekte Orientierung des Inserts (Anordnung in Transkriptionsrichtung) zum Promotor P_{bla} (Promotor von Gen bla, welches für die Ampicillinresistenz verantwortlich ist) eine Rolle.

### b) Insertion von beu in pKT240

pKT240 wurde mit PstI (1 Unit/µg) "linearisiert". Diese "linearisierte" DNA wurde mit dem 3 kb grossen PstI-Fragment (Insert) mit T4-DNA-Ligase (1 Unit/µg) im Ligationspuffer (20 mM Tris-HCl, pH 7,2, 10 mM DTT, 10 mM MgCl₂, 0,6 mM ATP) ligiert.
Die Ligation erfolgte über Nacht bei einer Temperatur von 12°C.
Zunächst wurde das erhaltene Ligationsgemisch nach der Methode von Lederberg und Cohen (J.Bacteriol., 119, 1974, S.1072-1074) in E.coli S.17-1 konjugiert. Die Selektion erfolgte auf NYB mit Kanamycin 25 µg/ml und gegen Ampicillin 100 µg/ml. Hybridplasmide mit dem Insert (Ampicillin-sensitiv, Kanamycin-resistent) in Trankriptionsrichtung zum Promotor P_{bla} erhielten die Bezeichnung pLO32.

### c) Konjugation von pLO32 in HK1349.4

Der konjugative Transfer von pLO32 aus E.coli S17-1 in HK1349.4 erfolgte ebenfalls nach der oben beschriebenen Methode. Die Selektion von HK1349.4 enthaltend Hybridplasmid pL032 erfolgte direkt auf die zu komplementierende Betain Verwertung (Mineralsalzmedium wie in Beispiel 1 enthaltend 0,2 Gew.% Betain).

### Beispiel 7:

### Stabilität von pLO32 in HK1349.4

Die Langzeitstabilität des Hybridplasmides in dem Mikroorganismus HK1349.4 wurde auf verschiedenen Medien getestet. Eindeutig erfolgte die Stabilisierung über die Verwertung der einzigen C-Quelle, Betain als Substrat (oder anderer Betaine, wie z.B. Cholin und Dimethylglycin) zu 100%.
Bei Stickstofflimitierung, wie in einem N-freien Medium in kontinuierlicher Recycling-Kultur ist auch eine Stabilisierung bei Nutzung von Betain als einzige N-Quelle, in Gegenwart weiterer C-Quellen, bis zu 100% nachgewiesen.

| MEDIUM | TOTAL ZELLEN | ZELLEN MIT PLASMID |
|---|---|---|
| MM (mit Ammoniumsulfat) | 100% | 5% |
| 0,2% Glc | | |
| (keine Selektion) | | |
| MM (ohne Ammoniumsulfat) | 100% | 20% |
| 0,1% Bet | | |
| 0,2% Glu | | |
| (keine Selektion) | | |
| MM (ohne Ammoniumsulfat) | 100% | 100% |
| 0,2% Bet | | |
| (C- und N-Quelle) | | |
| MM (mit Ammoniumsulfat) | 100% | 100% |
| 0,2% Bet | | |
| (C-Quelle) | | |
| MM (ohne Ammoniumsulfat) | 100% | 100% |
| 0,1% Bet | | |
| (N-Quelle) | | |
| 0,2% Glc | | |
| Abkürzungen der Tabelle: Glu = L-Glutamat (C- & N-Quelle) Glc = Glucose (C-Quelle) N = Stickstoff +, - Bet = Betaine (C- & N-Quelle) MM = Minimalmedium (Kulla et al., Arch. Microbiol. (1983), 135, S. 1 - 7 | | |

### Beispiel 8:

### Stabilität eines Hybridplasmides mit Biotransformations-Eigenschaften

Als Beispiel für die stabilisierende Wirkung des plasmidcodierten beu-Gens im beu-negativen Wirtstamm HK1349.4 wurde das Hybridplasmid pLO3 ausgewählt, welches bereits in der EP-A 0477 828 beschrieben ist.

Dabei handelt es sich um ein Hybridplasmid bestehend aus dem Vektor pKT240 und einem ClaI-HindIII-Fragment (2,35 Kb) des TOL-Plasmids, welches für die Gene xylMA codiert und unter die Kontrolle des Kanamycinphosphotransferase-Promotors kloniert wurde.

### a) Einführung einer neuen Kanamycin-Resistenz (Km^{R})

Die Kanamycin-Resistenzkasette (1,1Kb) aus pRME1 (Harayama et al., J. Bacteriol. 167 (1986), S. 455 - 461) wurde mit EcoRI (4 units (U) pro /µg DNA) herausgeschnitten und über Agarose Gelelektrophorese isoliert. 5'-überhängende Enden des DNA-Fragments wurden über Klenow-Raktion aufgefüllt (Current Protocols in Molecular Biology; John Wiley, New York 1987, Abschnitt 3.5). Die pLO3-DNA wurde mit HpaI ( 1 U pro µg DNA) geschnitten und mit 4,8 U alkalischer Phosphatase dephosphoryliert. Nach Isopropanol-Fällung wurde dieser "blunt end" geschnittene Vektor mit dem nunmehr "blunt end"-Insert (Km^{R}-Kasette) über Nacht bei 15°C ligiert. Ligationspuffer: 20 mM Tris, 10 mM MgCl₂, 0,6 mM ATP, pH 7,2, 10% PEG 6000, 0,5 U T4-DNA-Ligase.

E.coli K12 wurde mit dem Ligationsgemisch in analoger Weise zu Beispiel 2 transformiert und pLO3 (Km^{R}) enthaltende Transformanten wurden auf Nutrientagar mit 50 µg/ml Km selektioniert.

### b) Einführung des beu-Gens in pLO3 Km^{R})

Das Hybridplasmid pLO3 (Km^{R}) wurde mit PstI (1 U/µg DNA) "linearisiert" (vergl. Beispiel 6) und mit dem 3Kb grossen PstI-Fragments beu ligiert. Tansformation in E.coli und Konjugation in HK1349.4 erfolgte entsprechend zu Beispiel 6. Das Hybridplasmid erhielt die Bezeichnung pLOLO1 und entspricht pLO32 mit zusätzlicher Xylolmonooxygenase-Aktivität-Aktivität (xylMA).

c) Biotransformation mit stabilisiertem Hybridplasmid Agrobacterium/Rhizobium sp. HK1349.4/pLOLO1 wurde in Mineralsalzmedium (Arch. Microbiol. 135 (1983), S. 1 - 7) mit 0,2% Betain als einzige Kohlenstoffquelle bei 30°C angezogen. Die Biotransformation von 0,1% (v/v) 2,5-Dimethylpyrazin in 5-Hydroxymethyl-2-methylpyrazin wurde nachgeweisen.
Die Ausbeute an 5-Hydroxymethyl-2-methylpyrazin betrug nach 2 Tagen 20%.

### d) Stabilität von pLOLO1 in HK1349.4

Analog zu Beispiel 7 wurde die Langzeitstabilität des Hybridplasmids im Mikroorganismus HK1349.4 getestet. Sowohl über die Verwertung von Betain als einzige C-Quelle als auch als einzige N-Quelle konnte das Plasmid auch unter Biotransformationsbedingungen stabil im Produktionsstamm zu 100% erhalten werden.

## Patentansprüche

1. Mikroorganismen enthaltend
a) ein Hybridplasmid mit einem DNA-Fragment, enthaltend eine genetische Sequenz, die für die Verwertung von Betainen codiert und durch nachstehende Restriktionskarte (Fig. 1), charakterisiert ist 
PstI
3kb
SmaI
XhoI
SphI
PstI
Fig. 1
 und
b) eine Mutation im chromosomalen, für die Betain Verwertung codierenden Gen.

2. Mikroorganismen nach Patentanspruch 1 mit der Bezeichnung HK1349.4 enthaltend Hybridplasmid pLO32 (DSM-Nr. 6712).

3. Mikroorganismen nach Patentanspruch 1 oder 2, worin das Hybridplasmid zusätzlich die für Xylol-Monooxygenase codierenden Gene enthält.

4. Hybridplasmid bestehend aus dem DNA-Fragment nach Patentanspruch 1 und aus einem Expressionsvektor.

5. Hybridplasmid nach Patentanspruch 4 mit der Bezeichnung pLO32 bestehend aus dem DNA-Fragment nach Patentanspruch 1 und aus dem Expressionsvektor pKT240, wie hinterlegt in den Mikroorganismen mit der Bezeichnung HK1349.4 (DSM-Nr. 6712).

6. DNA-Fragment, enthaltend eine genetische Sequenz, die für die Verwertung von Betainen codiert und durch die Restriktionskarte in Patentanspruch 1 charakterisiert ist.

7. DNA-Fragment nach Patentanspruch 6 in Hybridplasmid pLO32, wie hinterlegt in den Mikroorganismen mit der Bezeichnung HK1349.4 (DSM-Nr. 6712).

8. Verfahren zur Herstellung von mit, bezüglich der Betain Verwertung, stabilen Plasmiden transformierten Mikroorganismen, dadurch gekennzeichnet, dass man
a) Betain verwertende Mikroorganismen im Chromosom derart mutiert, dass sie nicht mehr befähigt sind, Betain zu verwerten,
b) das isolierte, für die Betain Verwertung codierende DNA-Fragment nach Patentanspruch 6 in einen Expressionsvektor zu einem Hybridplasmid ligiert und
c) den in Schritt a) erhaltenen Mikroorganismus mit dem in Schritt b) erhaltenen Hybridplasmid transformiert und dann bezüglich Betain Verwertung selektioniert.

9. Verfahren nach Patentanspruch 8, dadurch gekennzeichnet, daß man
a) als Betain verwertenden Mikroorganismus den Mikroorganismus mit der Bezeichnung HK1349 (DSM 3944) im Chromosom derart mutiert, daß er nicht mehr befähigt ist, Betain zu verwerten,
b) das isolierte, für die Betain Verwertung codierende DNA-Fragment nach Patentanspruch 6 in den Expressionsvektor pKT240 ligiert und
c) den in Schritt a) erhaltenen Mikroorganismus mit dem in Schritt b) erhaltenen Hybridplasmid transformiert und dann bezüglich Betain Verwertung selektioniert.

10. Verwendung des DNA-Fragments nach Patentanspruch 6 zur Herstellung von bezüglich der Betain Verwertung stabilen Plasmiden.

## Claims

1. Micro-organisms containing
a) a hybrid plasmid having a DNA fragment, containing a genetic sequence coding for the utilisation of betaines and characterised by the restriction map shown below (Fig. 1), 
3kb
PstI
SmaI
XhoI
SphI
PstI
Fig. 1
 and
b) a mutation in the chromosomal gene coding for betaine utilisation.

2. Micro-organisms in accordance with claim 1, which have the designation HK1349.4 and contain hybrid plasmid pL032 (DSM No. 6712).

3. Micro-organisms in accordance with claim 1 or 2, wherein the hybrid plasmid additionally contains the genes coding for xylene-monooxygenase.

4. A hybrid plasmid, consisting of the DNA fragment in accordance with claim 1 and an expression vector.

5. The hybrid plasmid in accordance with claim 4, having the designation pLO32 and consisting of the DNA fragment in accordance with claim 1 and the expression vector pKT240, as deposited in the micro-organisms having the designation HK1349.4 (DSM No. 6712).

6. A DNA fragment containing a genetic sequence coding for utilisation of betaines and characterised by the restriction map in claim 1.

7. The DNA fragment in accordance with claim 6 in hybrid plasmid pLO32, as deposited in the micro-organisms having the designation HK1349.4 (DSM No. 6712).

8. A method for obtaining micro-organisms transformed by means of plasmides which are stable with respect to betaine utilisation, characterised in that
a) betaine utilising micro-organisms are mutated in the chromosome such that they cease to have the ability of utilising betaine,
b) the isolated DNA fragment coding for betaine utilisation in accordance with claim 6 is ligated into an expression vector to obtain a hybrid plasmid, and
c) the micro-organism obtained in step a) is transformed by means of the hybrid plasmid obtained in step b), and selection with respect to betaine utilisation is then performed.

9. The method in accordance with claim 8, characterised in that
a) as the betaine utilising micro-organism, the micro-organism having the designation HK1349 (DSM 3944) is mutated in its chromosome such as to cease having the ability of utilising betaine,
b) the isolated DNA fragment coding for betaine utilisation in accordance with claim 6 is ligated into the expression vector pKT240, and
c) the micro-organism obtained in step a) is transformed by means of the hybrid plasmid obtained in step b), and selection with respect to betaine utilisation is then performed.

10. Use of the DNA fragment in accordance with claim 6 for obtaining plasmides which are stable with respect to betaine utilisation.

## Revendications

1. Microorganismes contenant
a) un plasmide hybride avec un fragment ADN contenant une séquence génétique qui code pour l'utilisation de bétaïnes et qui est caractérisé par la carte de restriction suivante (figure 1), 
3kb
PstI
SmaI
XhoI
SphI
PstI
Fig. 1
 et
b) une mutation dans le gène chromosomique codant pour l'utilisation de bétaïne.

2. Microorganismes selon la revendication 1 avec la désignation HK1349.4 contenant le plasmide hybride pLO32 (DSM-n° 6712).

3. Microorganismes selon la revendication 1 ou 2, dans lesquels le plasmide hybride contient de plus les gènes codant pour xylène-monooxygénase.

4. Plasmide hybride constitué par le fragment d'ADN selon la revendication 1, et par un vecteur d'expression.

5. Plasmide hybride selon la revendication 4 portant la désignation pLO32 constitué par le fragment ADN selon la revendication 1 et par le vecteur d'expression pKT240, tel qu'il est déposé dans les microorganismes portant la désignation HK1349.4 (DSM n° 6712).

6. Fragment ADN contenant une séquence génétique qui code pour l'utilisation de bétaïnes et qui est caractérisé par la carte de restriction selon la revendication 1.

7. Fragment d'ADN selon la revendication 6 dans le plasmide hybride pL032, tel que déposé dans les microorganismes avec la désignation HK1349.4 (DSM n° 6712).

8. Procédé pour la préparation de microorganismes transformés par des plasmides stables, concernant l'utilisation de la bétaïne, caractérisé en ce que l'on procède aux étapes consistant à effectuer :
a) la mutation des microorganismes utilisant la bétaïne dans le chromosome de telle sorte qu'ils ne sont plus capables d'utiliser la bétaïne,
b) la ligation du fragment ADN codant pour l'utilisation de la bétaïne selon la revendication 6, dans un vecteur d'expression à un plasmide hybride, et
c) la transformation du microorganisme obtenu à l'étape a) avec le plasmide hybride obtenu à l'étape b) et sélectionner ensuite pour l'utilisation de la bétaïne.

9. Procédé selon la revendication 8, caractérisé en ce que l'on procède aux étapes consistant à effectuer :
a) la mutation en tant que microorganisme utilisant la bétaïne le microorganisme portant la désignation HK1349 (DSM 3944) dans le chromosome de telle sorte qu'il n'est plus capable d'utiliser la bétaïne,
b) la ligation du fragment d'ADN isolé codant pour l'utilisation de la bétaïne selon la revendication 6 dans le vecteur d'expression pKT240, et
c) la transformation du microorganisme obtenu à l'étape a) avec le plasmide hybride obtenu à l'étape b) et sélectionner ensuite pour l'utilisation de la bétaïne.

10. Utilisation du fragment d'ADN selon la revendication 6 pour la préparation de plasmides stables pour l'utilisation de la bétaïne.
